**Europäisches Patentamt**

(19) **European Patent Office**

**Office européen des brevets**

(11) Publication number : **0 511 978 B1**

# (12) EUROPEAN PATENT SPECIFICATION

(45) Date of publication of patent specification :
**23.03.94 Bulletin 94/12**

(51) Int. Cl.⁵ : **C07K 3/18,** C12N 15/62, C12P 21/06

(21) Application number : **91901985.1**

(22) Date of filing : **11.01.91**

(86) International application number :
**PCT/US91/00040**

(87) International publication number :
**WO 91/11454 08.08.91 Gazette 91/18**

(54) **METHOD OF PURIFYING RECOMBINANT POLYPEPTIDES.**

(30) Priority : **24.01.90 US 468724**

(43) Date of publication of application :
**11.11.92 Bulletin 92/46**

(45) Publication of the grant of the patent :
**23.03.94 Bulletin 94/12**

(84) Designated Contracting States :
**AT BE CH DE DK ES FR GB GR IT LI LU NL SE**

(56) References cited :
**EP-A- 0 157 235
EP-A- 0 163 573
EP-A- 0 244 147
EP-A- 0 327 522
WO-A-88/04692**

(73) Proprietor : **THE UPJOHN COMPANY
301 Henrietta Street
Kalamazoo, Michigan 49001 (US)**

(72) Inventor : **SHARMA, Satish, K.
7649 Hapton Oaks
Portage, MI 49081 (US)**
Inventor : **EVANS, David, B.
2028 Helen Street
Portage, MI 49002 (US)**

(74) Representative : **Perry, Robert Edward et al
GILL JENNINGS & EVERY Broadgate House 7
Eldon Street
London EC2M 7LH (GB)**

## Description

FIELD OF THE INVENTION

The present invention relates to a method of obtaining a purified desired protein product by producing it as a recombinant chimeric polypeptides which can be produced in a host, purified and cleaved into constitutive polypeptides.

BACKGROUND OF THE INVENTION

The rapid developments in recombinant DNA methodology have allowed the production of polypeptides, proteins, and their analogs in unlimited quantities in a very short period of time. These developments have created a need to handle purification of these proteins from complex mixtures in highly efficient and predictable manners.

Recombinant DNA technology may be used for the production of desired polypeptides and proteins in host cells. Genes for desired proteins may be isolated from the genetic material of cells which contain the gene in nature. The isolated gene may be inserted and expressed in host cell systems which produce protein products at high levels.

The desired protein products must then be isolated and recovered from the total amount of protein produced by the host cells. The purification of heterologous polypeptides produced by host cells can be very expensive and can cause denaturation of the protein product itself. An overview of protein purification techniques is provided in the Background Art section of U.S. Patent Number 4,782,137 issued Nov. 1, 1988 to Hopp et al. Among the methods to purify proteins that are described in Hopp, the most commonly practiced are ion-exchange, hydrophobic chromatography, and gel filtration. The major disadvantage of these approaches are the lack of specificity of each technique. Thus, these techniques are unsuitable to achieve pure protein in high yields. Even small changes in amino acid composition may change the purification properties. A modified purification procedure needs to be developed and optimized for each new protein. In the case of rDNA derived proteins, structural and functional consequences of heterologous gene expression (H. Bialy, Bio/technology, 5:884, 1987) are additional factors that may make it impossible to predict selection of these purification methods for a given protein.

Desired protein molecules may be isolated from complex mixtures by methods based on solubility differences. For example, isoelectric precipitation makes use of the alteration in protein solubility as a function of pH while fractionation with a solvent is based on the variation in protein solubility as a function of dielectric constant. Neutral salts, for example, ammonium sulfate, are employed to precipitate proteins due to decreased protein solubility based on high ionic strength of the salt. The drawback is that solvent fractionation can cause protein denaturation. Neither of these methods are capable of purifying proteins beyond a moderate level.

To avoid the negative elements of above techniques, affinity chromatography is often preferred. It is based on the ability of proteins to bind noncovalently but specifically with an immobilized ligand. When used alone, it can purify proteins from complex mixtures without significant loss. It requires the availability of the corresponding ligand for the desired protein. For example, an inhibitor for an enzyme or an antibody for a protein antigen. It should be stressed that in some cases it may be difficult to obtain a specific ligand and such ligands do not exist for all proteins. As a result, this technique has not been applied as a universal method for protein purification.

To circumvent this limitation, recombinant DNA technology may be used to provide an affinity purification system where a linker peptide may be used as an immunoaffinity ligand. This should provide a method that is capable of purifying recombinant proteins in a one-step, using affinity chromatography, without sacrificing high yields. Hopp relates to synthesis of a fusion peptide containing an antigenic linker peptide. The fusion peptide of Hopp is passed through a column containing immobilized antibodies which bind to the antigenic linker. Thus, the fusion peptide may be isolated. The major drawbacks of this technique are either the buffer conditions which are necessary to allow immunogenic complexing or the buffer conditions which must be present to terminate such complexes may denature the desired peptide product. Furthermore, the antigenic linker on the fusion peptide must be removed; an extra step involving addition of a protease and further purification. Finally, the cleavage by the endopeptidase is dependent upon the N-terminal of the desired protein.

Another problem in the area of recombinant proteins is the assay needed to determine the presence of the desired protein. Many proteins and peptides are not enzymes and, thus, their presence may only be determined by in vitro or in vivo biological methods. Purification strategies require a large number of highly accurate data and, therefore, bioassays are time consuming and tend to be inaccurate since a human protein may not work as well in animal models. Although immunoassays can be developed, this approach requires obtaining antisera for each protein. In addition, due to high specificity of antibodies, a small modification in the protein of interest may alter antibody specificity and, thus, reduce accuracy of the method.

The present invention is directed at the purification of biologically active recombinant polypeptides and/or proteins from bacterial or non-bacterial sources, most preferably those recombinant proteins expressed in a soluble form or secreted from the host as a fusion protein with an affinity peptide. According to the present invention, the desired protein is first produced as a fusion protein which, in addition to the amino acid sequence of the desired protein, contains a linker peptide. The linker peptide of the present invention contains a cleavage site recognized by an endopeptidase and is independent of the N-terminal sequence of the desired protein. When the fusion protein is produced, the desired protein may be isolated and purified by passing the fusion protein through a column containing immobilized endopeptidase. The fusion protein binds to the immobilized endopeptidase for a sufficient amount of time to allow it to be separated from other materials. Furthermore, the immobilized endopeptidase cleaves off the linker peptide, thereby producing pure desired protein.

## INFORMATION DISCLOSURE

EP 0 163 573 discloses DNA sequences encoding peptides cleavable by renin and for the insertion of said sequences into plasmid vectors for the production of cleavable fusion proteins. This document discloses the use of a DNA sequence for a renin cleavable linker to connect genes for two polypeptides to form a chimeric gene which encodes a fusion protein. When expressed, the fusion protein may be separated into the substituent polypeptides using renin. This document, however, neither teaches nor suggests the use of the renin cleavable linker in a one step purification and isolation method using immobilized renin columns to purify fusion peptides from crude supernatant and isolate the desired substituent polypeptide from the fusion protein.

European patent application number EP 244 147 relates to the purification of a desired protein by first producing it as a hybrid protein comprising a desired protein portion and a β-galactosidase moiety (BGM) portion linked by a renin cleavage site. The hybrid protein is immobilized in a column containing an affinity linker which recognizes and complexes with the BGM portion. Renin is added to the column, cleaving the hybrid and thereby liberating the desired protein from the column.

U.S. Patent No. 4,782,137 issued November 1, 1988 to Hopp et al., discloses the synthesis of a fusion protein having a highly antigenic N-terminal portion and a desired polypeptide at the C-terminal portion. According to Hopp et al., the fusion proteins are purified from crude supernatant by passing crude supernatant through a column containing immobilized antibodies which recognize the antigenic portion of the fusion protein. The immobilized antibodies immobilize the protein in the column while the undesired components of the supernatant are eluted. The column conditions can then be changed to eliminate the affinity conditions and cause the antigen-antibody complex to dissociate. The fusion protein is then eluted and collected. The antigenic N-terminal portion must then be removed from the C-terminal portion containing the desirable polypeptide to achieve a purified isolated desired polypeptide. In addition to the necessary second step, another drawback to Hopp et al. is that the conditions which allow for the affinity complexing and/or those which eliminate antibody-antigen recognition may cause denaturation of the fusion protein and loss of bioactivity of the desired protein.

U.S. Patent No. 4,751,180 issued June 14, 1988 to Cousens et al., discloses methods of producing a desired protein product by expression of a fusion gene. Cousens et al. disclose expression of a desired gene linked with a gene which encodes a protein product normally produced in a very large amount in the host. The two genes are linked with the DNA sequence which encodes a cleavable amino acid sequence. Thus, after the fusion protein is produced and purified, the desired protein may be separated from other protein at the cleavable linker.

Haffey, M.L. et al., DNA, Volume 6, 6:565-571 (1987), disclose a synthetic oligonucleotide that codes for an amino acid sequence specifically recognized and cleaved by renin. Haffey et al. teach that oligonucleotide may be inserted into a plasmid expression vector between two genes which encode desired proteins. Expression of the two genes linked with the oligonucleotide results in a fusion peptide which may be cleaved by renin. This publication corresponds to EP 0 163 573 described above.

Hopp, T.P. et al., Bio/Technol. 6:1204-1210, October 1988, disclose addition of an eight amino acid peptide to the N-terminus of a desired recombinant lymphokine in order to provide a antigenic N-terminus which can be used in immunoaffinity purification. This publication corresponds to U.S. Patent No. 4,782,137 described above.

European Patent Application Publication No. 0 244 147 relates to purification processes of hybrid proteins. A hybrid protein containing β-galactosidase moiety linked to a desired protein through a renin cleavage site is disclosed. The hybrid protein is purified from impurities using affinity chromatography. The affinity matrix binds to the β-galactosidase moiety. The desired protein is released from the hybrid protein by addition of renin to

EP 0 511 978 B1

the matrix whereby the desired protein is freed and the β-galactosidase moiety remains bound to the affinity matrix.

## SUMMARY OF THE INVENTION

The present invention provides a method of purifying a desired polypeptide comprising the steps of constructing a chimeric gene comprising a gene which encodes a desired polypeptide and which is fused with a DNA sequence that encodes an amino acid sequence cleavable by an endopeptidase, transforming suitable host cells with the chimeric gene in an expression system, producing the fusion polypeptide encoded by the chimeric gene; harvesting polypeptide products produced by the transformed cells, passing the products through a column containing immobilized endopeptidase and collecting desired polypeptide. Furthermore, the present invention provides a kit useful for purifying a desired polypeptide.

## DETAILED DESCRIPTION OF THE INVENTION

The present invention relates to the purification of biologically active recombinant proteins produced by transformed host cells. The desired biologically active recombinant proteins are most preferably produced in a soluble form or secreted from the host. According to the present invention, the desired biologically active protein is expressed as a fusion protein containing an affinity peptide that can be recognized by an enzyme capable of cleaving the protein at the recognition site. The fusion protein is immobilized and purified from the material present in the secretion media or extraction solution it is contained in and then processed to remove the affinity peptide portion molecule from the portion which comprises the desired protein. According to the present invention, a column is provided which contains an immobilized enzyme which recognizes immobilizes and cleaves the affinity peptide. Thus, the purification and processing steps take place in a single column wherein the impurities are eluted while the recombinant fusion protein is processed into the desired product.

The desired proteins which are produced by recombinant DNA technology and purified are expressed as a fusion protein. In addition to the desired protein, the fusion protein contains the affinity peptide, an amino acid sequence which is recognized by the immobilized enzyme as a cleavage site. The fusion protein is produced by host cells transformed with the genetic information encoding the fusion protein. The host cells may secrete the fusion protein into the culture media or store it in the cells whereby the cells must be collected and disrupted in order to extract the product.

The culture media containing the secreted fusion protein or the cell extracts containing the fusion protein are passed through the immobilized enzyme-containing column. All of the components of either solution freely pass through the column except the fusion protein. The immobilized enzyme recognizes the affinity peptide and impedes the movement of the fusion protein through the column. Thus, all the impurities are eluted through the column except the fusion protein. The immobilized enzyme cleaves the fusion protein at the scissile bond. This reaction requires a sufficient amount of time to allow the elution of all other components from the original solution. After the cleavage reaction occurs, the fusion protein is released as two components - the desired protein and the cleavage site-containing affinity peptide - which can be collected as a pure eluant. This pure eluant may be separated into its two components using simple techniques well known to those having ordinary skill in the art.

The present invention contains two components: a column containing an immobilized enzyme and a biologically active polypeptide or protein produced as a fusion protein containing an affinity peptide in a suitable host.

The terms "fusion protein" and "chimeric protein" as used herein are interchangeable and refer to polypeptides and proteins which consist of one or two linker peptides with affinity for enzymes which recognize cleavage sites on the linker and a biologically active polypeptide or protein or a short peptide linked directly or indirectly to the linker peptides.

The term "enzyme" referred to herein in the context of an immobilized enzyme and enzyme-containing column, means a polypeptide or protein which recognizes a specific amino acid sequence in a polypeptide and cleaves the polypeptide at the scissile bond. In the preferred embodiment of the present invention, human renin is the enzyme which is used in the immobilized enzyme column.

The term "human renin" referred to herein can be naturally occurring human renin or human renin produced from naturally occurring prorenin by activation, or recombinant human renin, or recombinant human renin obtained by activation of recombinant human prorenin. The recombinant human renin produced from the activation of recombinant human prorenin is the preferred human renin for immobilization of the fusion protein according to the present invention.

The terms "desired polypeptide" and "desired protein" as used herein are interchangeable and refer to the

4

polypeptide obtained after cleavage by the immobilized enzyme at the scissile bond.

The terms "linker peptide" or "affinity peptide" as used herein are interchangeable and refer to the amino acid sequence which is recognized and cleaved by the immobilized enzyme at the scissile bond.

The term "scissile bond" referred to herein is the juncture where cleavage occurs; for example the scissile bond recognized by human renin may be the Leu-Leu bond or the Leu-Val bond in the linker peptide or affinity peptide.

The present invention may be used to purify any prokaryotic or eukaryotic protein that can be expressed as the product of recombinant DNA technology in a transformed host cell. These recombinant protein products include hormones, receptors, enzymes, storage proteins, blood proteins, mutant proteins produced by protein engineering techniques, or synthetic proteins.

The chimeric proteins of this invention are prepared by recombinant DNA methodology. In accordance with the present invention, a gene sequence encoding a desired protein is isolated, synthesized or otherwise obtained and operably linked to a DNA sequence encoding the linker peptide. The DNA sequence encoding the linker peptide may be isolated from natural sources or synthesized using techniques well known by those having ordinary skill in the art. The hybrid gene containing the gene for a desired protein operably linked to a DNA sequence encoding a linker peptide is referred to as a chimeric gene.

The chimeric gene is inserted into an expression vector which allows for the expression of the desired chimeric protein in a suitable transformed host. The expression vector provides the inserted chimeric gene with the necessary regulatory sequences to control expression in the suitable transformed host.

There are six elements of control expression sequence for proteins which are to be secreted from a host into the medium, while five of these elements apply to chimeric proteins stored intracellularly. These elements in the order they appear in the gene are: a) the promoter region; b) the 5' untranslated region; c) signal sequence; d) the chimeric coding sequence; e) the 3' untranslated region; f) the transcription termination site. Fusion protein which are not secreted do not contain c), the signal sequence.

Methods and materials for preparing recombinant vectors and transforming host cells using the same, replicating the vectors in host cells and expressing biologically active foreign polypeptides and proteins are described in Principles of Gene Manipulation, by Old and Primrose, 2nd edition, 1981.

In addition to the genetic information necessary to encode and produce the fusion peptide, a column containing an immobilized enzyme is provided. This immobilized enzyme must retain its ability to recognize and cleave the specific amino acid sequence present in the linker peptide.

Additionally, the present invention relates to: genes which encode fusion proteins, expression vectors containing the same, microorganisms transformed with these expression vectors, and a process for obtaining these genes, expression vectors, and microorganisms transformed with said vectors.

In the preferred embodiment of the present invention, the linker peptides with affinity for human renin in accordance with this invention are defined by the general formula:

$$R1\text{-}Pro\text{-}Phe\text{-}His\text{-}Leu\text{-}Leu\text{-}Tyr\text{-}Tyr\text{-}Ser\text{-}R2$$

where R1 is a hydrogen atom, an amino acid, a sequence of few amino acids, a sequence of several amino acids, or a polypeptide. R2 represents a polypeptide or protein of interest.

Other suitable preferred linker peptides with affinity for human renin in accordance with this invention include peptides which comprise the following amino acid sequences:

$$Pro\text{-}Phe\text{-}His\text{-}Leu\text{-}Val\text{-}Ile\text{-}His\text{-}Ser;\ and,$$

$$Pro\text{-}Ile\text{-}Pro\text{-}Phe\text{-}His\text{-}Leu\text{-}Val\text{-}Ile\text{-}His\text{-}Ser.$$

In the preferred embodiment of the present invention, a column is provided that contains immobilized renin which recognizes and cleaves the above described linker peptides.

According to the present invention, the desired polypeptides produced may include tPA, IL-1, BST, IL-1 receptor, CD4, HIV RT and human nerve growth factor.

Conventions used to represent plasmids and fragments in Charts below, though unique to this application, are meant to be synonymous with conventional representations of plasmids and their fragments. Unlike the conventional circular figures, the single line figures on the charts represent both circular and linear double-stranded DNA with initiation or transcription occurring from left to right (5' to 3'). Asterisks (∗) represent the bridging of nucleotides to complete the circular form of the plasmids. Fragments do not have asterisk marks because they are linear pieces of double-stranded DNA. Endonuclease restriction sites are indicated below

5

the line. Genes and fragments are identified below the line.

## DESCRIPTION OF THE PREFERRED EMBODIMENTS

Example 1. IMMOBILIZATION OF RECOMBINANT HUMAN RENIN IN A COLUMN Biotinylation of renin:

In order to be able to immobilize active renin to a matrix, either the renin or the matrix has to be chemically modified to bind the other. In this example, the renin is chemically modified using a series of techniques revolving around the Biotin/Avidin system. That system is used because it is known to be useful in the immobilization of other enzymes while keeping them in an active form. Another reason is that the reagents used in the system are readily available from a number of sources.

Recombinant human renin is first biotinylated two different ways using two different protein modification reagents:

1. NHS-Biotin; and,
2. Sulfosuccimidobiotin.

These reagents are used to biotinylate the recombinant human renin as follows:

The human renin to be modified is first put into solution. Recombinant human renin is dissolved in water at a concentration of 2mg/ml. Reaction samples are prepared by combining .1ml aliquots of renin solution with .9mls of a 1mg/ml BSA solution dissolved in .1M $NaHCO_3$ pH 8.0 and dialyzed into solution of .1M $NaHCO_3$ overnight.

The human renin is modified by Reagent 1 using the following protocol. Reagent 1 is dissolved in DMSO at a concentration of 1mg/ml. A reaction sample of human renin is combined with .12mls of reagent/DMSO solution and reacted for 1hr at ambient temperature.

The human renin is modified by Reagent 2 using the following procedure. Reagent 2 is dissolved in dialysis buffer at a concentration 4.43mg/ml. To a renin sample, .057ml of the Reagent solution is added and reacted for 4hrs at ambient temperature.

Both reaction mixtures are separately then dialyzed extensively into 20mM NaH2PO4, 150mM NaCl, .1% Na Azide, pH 7.4. Modifications 1 and 2 both result in the maintenance of significant amounts of renin. Full activity is essentially maintained. The two modified renins demonstrate no loss in renin activity and allow for the immobilization these forms of renin on a solid matrix.

Immobilization of different biotinylated renins on Strepavidin Sepharose.

The biotinylated forms of recombinant renin produced above are attached to commercially prepared columns containing Strepavidin covalently bound to Sepharose. The interaction between biotin and avidin, although not covalent, has a dissociation constant on the order of 10-15 M. The modified renin is attached to the avidin through this affinity without loss of renin activity. The strepavidin form of avidin is used because it is reported to have lower non-specific binding of proteins than avidin.

The two active derivatives of renin are dialyzed into PBS/TWEEN (20mM NaH2PO4, 150mM NaCl, .2% TWEEN pH 7.5). One ml samples of biotinylated renin were loaded onto separate .5ml columns of strepavidin Sepharose which has been equilibrated in PBS/TWEEN. The columns are then washed with PBS minus TWEEN and fractions which do not bind to the columns are assayed for renin activity using the standard renin activity assay.

In both cases, less than .01% of the modified renin applied to the column does not bind to the column. Thus, the biotinylated forms of renin not only retain renin activity but are capable of binding strepavidin. The renin which bound the strepavidin column following biotinylation bound specifically through the biotin moiety. The chemically modified recombinant human renin is immobilized in a solid support. Two different columns are produced.

Hydrolysis of renin substrate on immobilized renin columns

To show that the renin still has activity even in a bound state, purified human angiotensinogen is passed over the columns and the renin cleavage product is measured in the standard ANG-I RIA. Samples of human renin substrate are dialyzed into 150mM Na2HPO4, 160mM NaCl, 3mM EDTA, pH 6.0 and 2.5mls of a 5µg/ml solution of angiotensinogen is chromatographed on both renin columns by passing the angiotensinogen over the columns three times and collecting the material which does not bind. Columns used are .5mls and equilibrated with the buffer described above. The chromatography is done at ambient temperature under gravity.

The columns are then washed with the starting buffer and fractions are collected. All the fractions are assayed for the presence of ANG-I using the RIA and the total amount of ANG-I collected is compared to the total ANG-I expected. The expected amount of ANG-I is determined by incubating a 50ul aliquot of the pre-column angiotensinogen with an excess amount of renin at 37 C for 2hrs.

The immobilized renin which had been derivatized using either NHS-Biotin or Sulfosuccinimidobiotin is essentially fully active toward renin substrate under the conditions described above. Both of these columns completely cleave substrate, indicating that the renin which had been immobilized is still active and capable of hydrolyzing substrate.

Example 2 CONSTRUCTION OF A FUSION PEPTIDE CONTAINING AN AFFINITY PEPTIDE AND HIV RT

There are basically three known properties of human renin that used in conjunction, for the immobilization of chimerics containing renin-specific handle:

a) Much slower hydrolysis of non-primate substrates at its optimum pH;

b) Slower catalysis when used in an environment with a pH slightly higher than the optimal pH;

c) Retention of activation at 4° C; similar to many other proteins which undergo inactivation at higher temperatures.

Once biologically active human renin has been immobilized it is possible to immobilize recombinant proteins (enzymes, hormones, and receptors) containing a linker peptide at the N-terminal. The catalytic rate of cleavage in the presence of human renin is highly species dependent with respect to substrate used. The N-terminal side of the scissile bond (1) from many species are identical while differences in specific amino acid residues on the C-terminal side of scissile bond are different and are known to affect hydrolysis of various substrates by human renin. For example, rat plasma angiotensinogen is hydrolyzed by human renin 25-fold slower than human angiotensinogen.

Asp-Arg-Val-Tyr-Ile-His-Pro-Phe-His-Leu-Val-Ile-His- Human

Asp-Arg-Val-Tyr-Ile-His-Pro-Phe-His-Leu-Leu-Tyr-Tyr- Rat

-----------------N-terminal----------- ↑ ---C-terminal----

Fusion proteins prepared by linking DNA encoding for the linker peptide in frame with a gene for heterologous protein are used for immobilization of fusion proteins with renin columns.

For example:

Pro-Ile-Pro-Phe-His-Leu-Leu-Tyr-Tyr-Ser-RT;

wherein RT is a desired protein.

Construction of Oligonucleotides Which Are Used to Construct Gene Sequences That Encode Affinity Peptides and HIV RT

The following oligonucleotides (1-6) which are used to construct linker sequences of construct #1 (Pro-Ile-Pro-Phe-His-Leu-Val-Ile-His-Ser-) and construct #2 (Pro-Ile-Pro-Phe-His-Leu-Leu-Tyr-Tyr-Ser-) are synthesized separately using standard methods.

1. 5' ATT GAG ACT GTA CCA GTA AAA TTA AAG CCA GGA ATG GAT GGC

CCA

AAA GTT AAA CAA TGG 3'

2. 5' CCA TTG TTT AAC TTT TGG GCC ATC CAT TCC TGG CTT TAA TTT TAC

TGG TAC AGT CTC AAT AGG G 3'

3. 5' AA TTC ATG CCC ATT CCC TTT CAC TTA GTA ATT CAC AGC CCC ATT

AGC CCT 3'

4. 5' CT AAT GGG GCT GTG AAT TAC TAA GTG AAA GGG AAT GGG CAT G

3'

5. 5' AA TTC ATG CCC ATT CCC TTT CAC TTA CTA TAC TAC AGC CCC ATT

AGC CCT 3'

6  5' CT AAT GGG GCT GTA GTA TAG TAA GTG AAA GGG AAT GGG CAT G

3'

Polyacrylamide gels are used to purify these oligonucleotides. The purified oligonucleotides are annealed and ligated in the following formats.

Construction #1

5'---oligo #3-------3' 5'---oligo #1---3'

3'-oligo #4--5' 3'--oligo #2--5'

Construction #2

5'---oligo #5-------3' 5'---oligo #1---3'

3'-oligo #6---5' 3'--oligo #2--5'

The above constructions contain the following restriction sites:

$$5\text{'EcoR1----------------}3\text{'}$$
$$3\text{'---------------}5\text{'Bal I}$$

The above EcoR1/Bal I pieces are ligated into the final vector as 1 part of a three-way ligation. The other two pieces for this ligation are obtained as follows.

Construction of DNA Expression Vectors Which Contain Affinity Peptide DNA Sequences Fused To HIV Reverse Transcriptase RT Gene

The gene for HIV reverse transcriptase is widely available and the methods used to insert restriction enzyme sites at its flanking regions are well known to those having ordinary skill in the art. In this example, a clone, TM-3, described by Diebel, M. R. et al, AIDS and Human Retroviruses, In Press (1990), which contains the gene for HIV reverse transcriptase, RT is cut with Bal I/Hind III to release the 1.5Kb HIV RT gene fragment.

Plasmid pKK223-3 (Pharmacia) is cut with Eco R1/Hind III and the 4.55Kb fragment was isolated.

The two desired fragments are purified on agarose gels and mixed with either of the two ligated sets of oligos for construction # 1 and Construction # 2. These are ligated to the final plasmid to yield HIV RT containing the linker at its N-terminal. The structure of a recombinant chimeric (linker-HIV RT) expression vector is shown below.

```
     EcoR1   Bal1        HindIII

       ↓       ↓           ↓

 5' *__|_____|___1.5kb___|_____4.55kb_____  *   3'
      OLIGOS   RT from TM-3   fragment from pKK223-3
```

Production of Fusion Proteins

The plasmids are transformed into the JM-109 strain of E. coli using standard protocols. Cells were grown on ampicillin containing plates. Clones containing reverse transcriptase (RT) activity are selected and sequenced using $^{32}P$ dATP. Colonies containing the correct DNA sequence are grown for expression of the chimeric genes. For preparation of the chimeric proteins for purification in accordance with the current invention, approximately 3g of E. coli cell paste is suspended in 30mls 0.25M potassium phosphate, pH 7.2 containing 1mM dithiothreitol (DTT), EDTA, phenylmethylsulfonyl fluoride (PMSF), and benzamidine HCL, 10mg/liter aprotinin, leupeptin, and bestatin. This suspension is passed through a French Press three times to break the cells. Cell lysates are centrifuged at 12,000 rpm for 1hr. The HIV RT is detached from the fusion protein and isolated from the crude supernatant using immobilized human renin.

Example 3 CONSTRUCTION OF A FUSION PEPTIDE CONTAINING A MODIFIED ANGIOTENSIN LINKER AND tPA

Construction of Plasmid for Extended (Ser-Tyr), Modified AngI (+3, Val-Ile-His)-tPA

This example uses the sib transcriptional terminator as a regulatory sequence for terminating transcription of the fusion protein gene comprising the tPA gene linked to a modified angiotensin linker. The sib transcription terminator is isolated from bacteriophage lambda. The methods performed to accomplish this are well know to those having ordinary skill in the art using readily available materials (See Weighous, T. F. and W. G. Tarpley, Biochem. and Biophys. Res. Comm., Vol. 143, 2:593-599, 1987).

The Hind III site of pGEM-4 (Promega) is replaced with a Sal 1 linkers (New England Biolabs).

The sib transcriptional terminator is inserted as an Xho fragment into the Sal 1 site of a modified pGEM-4.

A 473bp piece from the 5' end of the tPA gene is obtained as follows. The full length tPA cDNA is cleaved with Hga1, releasing a 517 bp Hga1 fragment. Termini are made flush with Klenow and BamH1 linkers are added to the termini using standard procedures. The modified fragment is then digested with BamH1 and Nar1 to produce a 440 bp BamH1 - Nar1 fragment representing the 5' portion of the tPA sequence beginning with a BamH1 site at nucleotide number 78 (numbering according to Pennica et al.). This fragment was subsequently assembled with the 3' portion of the gene represented by a Nar1-Bg1II fragment isolated from the full length tPA cDNA and the vector pKC7 which is digested with BamH1 and Bg1II, yielding plasmid pPSA. This plasmid contains a version of the tPA gene which contains an engineered BamH1 site at the 5' end (78) throughout the full 3' non-coding portion of the gene.

The 473bp fragment is then inserted as a Bam H-1/Nar I fragment 3' to the terminator.

The oligonucleotides corresponding to the ANG-I(+3) linker are reconstructed from 4 oligonucleotides; the 29-mer GATCTTACGACATAGTGTACATACACCCC and the 37-mer TTCCACCTCGTCATCCACTCTTACCAAGTGATCTGCA and their complementary oligonucleotides. These oligos are purified, kinased, annealed and repurified according to standard methods and then inserted as a Bgl II/Pst I fragment into the 5' end of the tPA gene.

The construct is sequenced by the dideoxy method to ensure correct assembly. The remaining 3'-end of the tPA gene is isolated as a Nar I/Bam H1 fragment from ptPA-IPA and ligated to the purified 5' Bam HJ-1/Nar fragment of DNA which contains the oligonucleotides for ANG-I(+3).

The full length tPA gene containing the oligonucleotides is gel purified and inserted as a Bam H1 fragment into the Bg1 II site in the TFW-9 vector.

The BPV genome necessary for expression in mammalian cells is then added as a Bam H-1 fragment and the entire construct was used to transfect C-127 cells as described.

Culturing of C-127 Murine Cells

Murine C-127 cells are obtained from the American Type Culture Collection and grown in Dulbecco's modified Eagle's minimal essential media DMEM (Gibco Cat.#320-1965) containing 10% fetal calf serum and 100 units/ml Penicillin and 100 μg/ml streptomycin. Cells were transfected by standard procedures using 5μg DNA. Three weeks later, transformed foci are picked and expanded as individual cell lines. The individual cell lines are grown and assayed for secreted tPA activity. Cell line DE-9-22 was found to contain the most secreted tPA activity and was chosen for further expansion.

Cells are then grown in the above serum-containing media containing 1 μg/ml aprotinin and 10 mM EACA and expanded to 100 mm plates. These cells are then passed and seeded on Cytodex 1 microcarrier beads (Pharmacia) in 3,000 ml spinner flasks at a $6.3 \times 10^7$ cells/ml and grew to a density of $1.0 \times 10^9$ cells/ml (after nine days). Once the cells reach maximum density the culturing protocol is modified as follows. Cells are rinsed three times with an isotonic buffer and placed in serum-free media which consists of equal parts DMEM 320-1885 (Gibco) and MCDB Medium 301 Formula 78-0037AJ (Gibco) supplemented with 10 mM Hepes, 5 μg/ml insulin, 5 μg/ml transferrin, 5 ng/ml sodium selenite and 4 mM L-glutamate. There may also be some residual serum present in the medium. The media also contains antibiotics and protease inhibitors. The media may be harvested and the components of the fusion protein recovered by passing the lysates through an immobilized renin column.

Example 4 PROCEDURE FOR COLLECTING FUSION PROTEINS USING IMMOBILIZED HUMAN RENIN COLUMN

Lysates from E. coli expressing the recombinant chimerics or conditioned media into which the chimeric has been secreted by mammalian cells, are added to the immobilized renin column at a pH 7.0 which is slightly higher than the optimum pH of human renin. The column is allowed to remain at 5°C. Recombinant proteins to be used as a starting material includes purified products, partially purified proteins obtained during purification process or crude extract containing the desired recombinant protein.

It is recommended that the expressed polypeptide be in soluble form, preferably in the absence of denaturants. The use of a buffer solution compatible with the column equilibration buffer is recommended, to avoid inhibition of immobilized human renin. Binding pH is in the range 5 to 8.5; from the point of view of stability of both immobilized human renin and the desired recombinant protein. A pH range of about 5.5 to 7.5 is especially preferable. Binding time ranges from about 30 minutes to 50 hrs. Binding temperature ranges from 5 C to 37 C, more preferably 5 C for chimerics containing the human Leu-Val scissile bond and 25 C for chimerics containing the rat Leu-Leu scissile bond.

Since immobilized form of human renin is employed for purification of recombinant proteins, the enzyme is used repeatedly and therefore the method is economical and efficient.

As hosts, both E. coli and mammalian cells are preferred hosts. Of these two, E. coli is the preferred host according to this invention.

**Claims**

1. A method of purifying a desired polypeptide, comprising the steps of:

   a) constructing a chimeric gene comprising a gene which encodes the desired polypeptide and which is fused with a DNA sequence which encodes an amino-acid sequence cleavable by an endopeptidase;

   b) transforming suitable host cells with the chimeric gene in an expression system such that said chimeric gene is expressed, producing a fusion polypeptide encoded thereby;

   c) harvesting polypeptide products produced by the transformed cells and passing said products through a column containing immobilised endopeptidase; and

   d) collecting the desired polypeptide.

2. A method according to claim 1, wherein the endopeptidase is renin.

3. A method according to claim 2, wherein the amino-acid sequence comprises:

   ```
   Pro-Phe-His-Leu-Leu-Tyr-Tyr-Ser;
   Pro-Ile-Pro-Phe-His-Leu-Leu-Tyr-Tyr-Ser; or
   Pro-Phe-His-Leu-Val-Ile-His-Ser.
   ```

4. A method according to claim 3, wherein the amino-acid sequence comprises Pro-Phe-His-Leu-Leu-Tyr-Tyr-Ser.

5. A method according to any preceding claim, wherein the desired polypeptide is tPA, IL-1, BST, IL-1 receptor, CD4, HIV RT or human nerve growth factor.

6. A kit for use in purifying a desired polypeptide, comprising

   a) a DNA molecule comprising a gene which encodes the desired polypeptide fused with a DNA sequence as defined in any of claims 1 to 4; and

   b) a column containing immobilised endopeptidase, capable of harvesting the polypeptide product.

7. A kit according to claim 6, wherein the desired polypeptide is as defined in claim 5.


**Patentansprüche**

1. Verfahren zum Reinigen eines gewünschten Polypeptids, umfassend folgende Stufen:

   a) Konstruieren eines chimären Gens, umfassend ein Gen, welches für das gewünschte Polypeptid kodiert und mit einer DNS-Sequenz mit Kodierung für eine durch eine Endopeptidase spaltbare Aminosäuresequenz verschmolzen ist;

   b) Transformieren geeigneter Wirtzellen mit dem chimären Gen in einem Expressionssystem, derart, daß das chimäre Gen unter Bildung eines dadurch kodierten Fusionspolypeptids exprimiert wird;

   c) Ernten der durch die transformierten Zellen gebildeten Polypeptidprodukte und Hindurchleiten der Produkte durch eine immobilisierte Endopeptidase enthaltende Säule und

   d) Sammeln des gewünschten Polypeptids.

2. Verfahren nach Anspruch 1, wobei die Endopeptidase aus Renin besteht.

3. Verfahren nach Anspruch 2, wobei die Aminosäuresequenz

   ```
   Pro-Phe-His-Leu-Leu-Tyr-Tyr-Ser
   Pro-Ile-Pro-Phe-His-Leu-Leu-Tyr-Tyr-Ser; oder
   Pro-Phe-His-Leu-Val-Ile-His-Ser umfaßt.
   ```

4. Verfahren nach Anspruch 3, wobei die Aminosäuresequenz Pro-Phe-His-Leu-Leu-Tyr-Tyr-Ser umfaßt.

**5.** Verfahren nach einem der vorhergehenden Ansprüche, wobei das gewünschte Polypeptid aus tPa, IL-1, BST, IL-1 Receptor, CD4, HIV RT oder menschlichen Nervenwachstumsfaktor besteht.

**6.** Kit zur Verwendung bei der Reinigung eines gewünschten Polypeptids, umfassend
a) ein DNS-Molekül mit einem Gen mit Kodierung für das gewünschte Polypeptid, das mit einer DNS-Sequenz gemäß einem der Ansprüche 1 bis 4 verschmolzen ist, und
b) eine Säule mit immobilisierter Endopeptidase mit der Fähigkeit zur Ernte des Polypeptidprodukts.

**7.** Kit nach Anspruch 6, wobei das gewünschte Polypeptid gemäß Anspruch 5 definiert ist.


**Revendications**

**1.** Procédé de purification d'un polypeptide désiré, comprenant les étapes :
a) de construction d'un gène chimérique comprenant un gène qui code pour le polypeptide désiré et qui est fusionné avec une séquence d'ADN qui code pour une séquence d'amino-acides clivable par une endopeptidase ;
b) de transformation des cellules-hôtes convenables avec le gène chimérique dans un système d'expression de telle sorte que ledit gène chimérique soit exprimé, en produisant un polypeptide de fusion codé ainsi par ce gène ;
c) de collecte des produits polypeptidiques formés par les cellules transformées et de passage desdits produits à travers une colonne contenant une endopeptidase immobilisée ; et
d) de séparation du polypeptide désiré.

**2.** Procédé suivant la revendication 1, dans lequel l'endopeptidase est la rénine.

**3.** Procédé suivant la revendication 2, dans lequel la séquence d'amino-acides comprend :

```
Pro-Phe-His-Leu-Leu-Tyr-Tyr-Ser ;
Pro-Ile-Pro-Phe-His-Leu-Leu-Tyr-Tyr-Ser ; ou
Pro-Phe-His-Leu-Val-Ile-His-Ser.
```

**4.** Procédé suivant la revendication 3, dans lequel la séquence d'amino-acides comprend Pro-Phe-His-Leu-Leu-Tyr-Tyr-Ser.

**5.** Procédé suivant l'une quelconque des revendications précédentes, dans lequel le polypeptide désiré est le tPA, la Il-1, le BST, le récepteur de IL-1, le CD4, le HIV RT, ou le facteur de croissance neurale humain.

**6.** Kit destiné à être utilisé dans la purification d'un polypeptide désiré, comprenant
a) une molécule d'ADN comprenant un gène qui code pour le polypeptide désiré fusionné avec une séquence d'ADN répondant à la définition suivant l'une quelconque des revendications 1 à 4 ; et
b) une colonne contenant une endopeptidase immobilisée, capable de séparer le produit polypeptidique.

**7.** Kit suivant la revendication 6, dans lequel le polypeptide désiré répond à la définition suivant la revendication 5.